# EUROPEAN PATENT SPECIFICATION

(11) **EP 3 124 969 B1**
(45) Date of publication and mention of the grant of the patent: **01.05.2019**
(21) Application number: 15770205.1
(22) Date of filing: 23.03.2015
(51) Int. Cl.: G01N 33/53, G01N 21/64, G01N 33/48, G01N 33/536, A61B 5/00, G01N 33/58, G01N 33/68

(54) **BIOLOGICAL-MATERIAL QUANTITATION METHOD BASED ON MULTIPLE-ANTIGEN IMMUNOSTAINING**
QUANTIFIZIERUNGSVERFAHREN VON BIOLOGISCHEM MATERIAL AUF DER BASIS VON MULTI-ANTIGEN-IMMUNFÄRBUNG
PROCÉDÉ D'ANALYSE QUANTITATIVE DE MATÉRIAU BIOLOGIQUE SUR LA BASE D'UNE IMMUNOCOLORATION À ANTIGÈNES MULTIPLES

(30) Priority: 24.03.2014 JP 2014059883
(43) Date of publication of application: 01.02.2017
(73) Proprietor: Konica Minolta, Inc., Tokyo 100-7015 (JP)
(72) Inventor: GOUDA, Hideki, Tokyo 100-7015 (JP); ISODA, Takeshi, Tokyo 100-7015 (JP); WATANABE, Yasuhiro, Tokyo 100-7015 (JP); GONDA, Kohsuke, Sendai-shi Miyagi 980-8577 (JP); OHUCHI, Noriaki, Sendai-shi Miyagi 980-8577 (JP); WATANABE, Mika, Sendai-shi Miyagi 980-8577 (JP)
(74) Representative: Gille Hrabal
(86) International application number: PCT/JP2015/058703
(87) International publication number: WO 2015/146896

(56) References cited:
- WO-A1-2011/042874
- WO-A1-2012/029342
- WO-A1-2012/029752
- WO-A1-2013/146841
- WO-A1-2013/151511
- WO-A1-2015/045962
- JP-A- 2008 268 167
- JP-A- 2009 536 355
- JP-A- 2013 057 631
- JP-A- 2013 057 631
- JP-B2- 5 039 264
- US-A1- 2002 076 729
- US-A1- 2006 160 168
- US-A1- 2009 042 238
- DANIEL W PRG A[1/4]STNER ET AL: "Spatiotemporal analysis of endocytosis and membrane distribution of fluorescent sterols in living cells", HISTOCHEMISTRY AND CELL BIOLOGY, SPRINGER, BERLIN, DE, vol. 130, no. 5, 12 September 2008 (2008-09-12), pages 891-908, XP019657759, ISSN: 1432-119X, DOI: 10.1007/S00418-008-0488-6
- IRIE ET AL: "Recruitment of Alix/AIP1 to the plasma membrane by Sendai virus C protein facilitates budding of virus-like particles", VIROLOGY, ELSEVIER, AMSTERDAM, NL, vol. 371, no. 1, 29 October 2007 (2007-10-29), pages 108-120, XP022417956, ISSN: 0042-6822, DOI: 10.1016/J.VIROL.2007.09.020
- POUWELS P H ET AL: "LACTOBACILLI AS VEHICLES FOR TARGETING ANTIGENS TO MUCOSAL TISSUES BY SURFACE EXPOSITION OF FOREIGN ANTIGENS", METHODS IN ENZYMOLOGY, ACADEMIC PRESS, US, vol. 336, 1 January 2001 (2001-01-01), pages 369-389, XP001012600, ISSN: 0076-6879, DOI: 10.1016/S0076-6879(01)36602-8

## Description

### Technical Field

The present invention relates to a method for quantifying a biological material (target biological material) expressed on a cell membrane of tissue sections. More specifically, the present invention relates to a method for quantifying target cells using a multiple immunostaining method in which a target biological material and a biological material (reference biological material) other than the target biological material are immunostained with fluorescent labels, respectively.

### Background Art

Pathological diagnosis includes observing, with a microscope, materials collected from human bodies (samples prepared from specimens such as cells or tissues) to determine the presence or absence of lesions and the type of lesions by using pathological knowledge and techniques. Pathological diagnosis is performed in clinical practice to determine therapeutic and surgical plans.

A technique widely used for such pathological diagnosis is staining based on immunohistochemistry (IHC), in which a complex of a label, a specific disease-related molecule (antigen), and an antibody to the molecule is formed in order to observe how the molecule is expressed in samples. For example, HER2 (human epidermal growth factor receptor), which can be overexpressed on the cell membrane of breast cancer cells, is a membrane protein that can be targeted by a molecular target drug Herceptin (registered trademark) (generic name: trastuzumab). The determination of whether or not overexpression of HER2 is observed in breast cancer tissues based on staining images is an important index for predicting the therapeutic effect of this molecular target drug.

Immunohistochemical staining traditionally uses a certain technique (dye staining technique) in which an enzyme capable of reacting with a specific substrate to develop a color is used as a marker. For example, DAB staining is a dye staining method using peroxidase as a marker enzyme and diaminobenzidine (DAB) as a substrate, in which their enzyme reaction causes an antigen-containing site to develop a brown color observable in the bright field (optical microscope). Unfortunately, DAB staining and other dye staining methods using an enzyme marker have a problem in that the staining concentration significantly depends on environmental conditions such as temperature and time so that the actual antibody expression level is difficult to estimate from the staining concentration.

In recent years, therefore, other techniques (fluorescent labeling techniques) using fluorescent labels instead of enzyme markers and capable of allowing antigens to appear as bright spots of fluorescence observable in the dark field (fluorescence microscope or confocal laser microscope) have come into use in immunohistochemical staining.

For example, Patent Literature 1 proposes a method that includes labeling a biological material on the cell membrane with inorganic semiconductor nanoparticles or particles containing a fluorescent organic dye or inorganic semiconductor nanoparticles (fluorescent material-containing nanoparticles) while labeling the cell membrane itself with a fluorescent material capable of non-specifically binding to the cell membrane, such as eosin or PKH dye (trade name: CellVue (registered trademark), having a moiety capable of emitting strong fluorescence and a long lipophilic tail bonded thereto) and quantifying the biological material on the cell membrane from the resulting fluorescence observation image.

Patent Literature 2 proposes a method that includes using antibodies labeled with different fluorescent dyes to stain a target protein to be quantified and a reference protein, respectively, in a sample, measuring the total fluorescence intensity of each of the stained target and reference proteins, and using the ratio of the total fluorescence intensity of the target protein to the total fluorescence intensity of the reference protein as a value for quantifying the target protein. In the method described in Patent Literature 2, examples of the target protein include a variety of proteins specifically expressed in cancer cells, such as proteins as anticancer drug sensitivity markers, proteins related to the side effects of anticancer drugs, proteins as prognosticator markers, and key proteins for diagnosis (generally secretory proteins) . On the other hand, examples of the reference protein include proteins encoded by house-keeping genes (house-keeping proteins), constantly expressed in the cytoplasm, such as β-actin and GAPDH (glyceraldehyde-3-phosphate dehydrogenase).

### Citation List

### Patent Literatures

Patent Literature 1: JP 2013-057631 A
Patent Literature 2: JP 2008-268167 A

### Summary of Invention

### Technical Problem

Sections of cells, tissues, organs, or other parts (pathological samples) for use in pathological diagnosis vary in their preserved condition or in the degree of denaturation of proteins in the samples, depending on the time until the fixation in the sample preparation step, the fixation time, and other factors. The antibody recognition rate in an antigen-antibody reaction varies with the degree of denaturation, so that in some cases, a constant result cannot be obtained even when samples are prepared with the same tissues or cells. In addition, even with the same expression level of the target protein, the amount of the protein detected on a planar staining image can vary with the thickness of the section obtained by slicing the fixed sample (the thicker the section, the more the detected amount). Therefore, there is a problem in that the value quantified based on a fluorescent material such as fluorescent material-containing nanoparticles can vary also in the observation of a plurality of samples obtained by slicing tissues that are considered to have substantially the same expression level of the target protein.

In addition, pathological diagnosis for estimating the effect of a molecular target drug requires only a target protein on the cell membrane to be accurately quantified. To meet this requirement, it is necessary to extract the signal derived from the fluorescent material specifically binding to the target protein on the cell membrane and to eliminate, as much as possible, the signal derived from the fluorescent material non-specifically adsorbed on materials other than the target protein in the cytoplasm. Unfortunately, the method of measuring the total fluorescence intensity as described in Patent Literature 2 is not provided with any means for identifying the position of the target protein. In this method, therefore, the signals derived from all the fluorescent materials in the pathological sample, which include the fluorescent material non-specifically adsorbed as mentioned above, are used for the quantification, so that only the target protein on the cell membrane cannot be accurately quantified.

In addition, the size of cells in cancer tissues differ from specimen to specimen, and the relative area of the cell membrane region (relative to the cytoplasm region) in a staining image can be smaller in the case of large cells than in the case of small cells. Therefore, when the ratio of the total fluorescence intensity derived from the target protein mainly on the cell membrane to the total fluorescence intensity derived from a house-keeping protein as a reference protein in the cytoplasm is calculated as an evaluation value as described in Patent Literature 2, the evaluation value can be lower in the case of large cells than in the case of small cells even when the expression levels of the target protein per unit area of the cell membrane are the same in both cases.

Also in the invention described in Patent Literature 2, the house-keeping protein used as the reference protein exists mainly in the cytoplasm whereas the target protein exists mainly on the cell membrane. During sample preparation, the cytoplasm part and the cell membrane part are degraded differently. Therefore, the ratio of the total fluorescence intensity derived from the target protein on the cell membrane to the total fluorescence intensity derived from the house-keeping protein as the reference protein in the cytoplasm is influenced by the difference in the degraded state, which means that the expression level of the target protein on the cell membrane cannot be accurately evaluated regardless of the degraded state of the cell membrane part.

In addition, different cancer cells have different properties, and even a house-keeping protein is not expressed at a constant level and can significantly vary in expression level. Patent Literature 2 specifically discloses only an embodiment in which only a single house-keeping protein is used as the reference protein. In the decision of the suitability of molecular target drugs, such an embodiment may fail to make constant evaluation for specimens collected from a wide range of patients, which can include specimens significantly different in the expression level of the house-keeping protein from normal specimens.

On the other hand, in the method described in Patent Literature 1, a material capable of non-specifically binding to the cell membrane is used to stain the cell membrane. In this method, the degraded state brought about during sample preparation also have different influences on the staining with the non-specific binding material and on the staining of a biological material (target protein) on the cell membrane based on specific binding caused by the antigen-antibody reaction. Patent Literature 1 discloses that only the fluorescence with which a biological material on the cell membrane is labeled is extracted using the fluorescent staining of the cell membrane and the biological material is quantified using the extracted fluorescence. However, Patent Literature 1 does not disclose any technical idea for correcting a fluorescence signal (brightness or the number of bright spots) from a label on a biological material on the cell membrane by using a fluorescence signal (brightness) from the stained cell membrane.

The article "LACTOBACILLI AS VEHICLES FOR TARGETING ANTIGENS TO MUCOSAL TISSUES BY SURFACE EXPOSITION OF FOREIGN ANTIGENS", METHODS IN ENZYMOLOGY, Volume 336, Pages 369-389; 20010101; POUWELS P H; ET AL discloses a method of detection and quantification of antigen expression on lactobacilli cells using flow cytometric techniques. The method detects and quantifies antigens expressed on the cell surface of (gram-positive) bacteria. For the detection of the antigen, immunofluorescent staining with a specific antibody directed against the antigen of interest was used. In order to quantify the amount of fluorescing probe, which is directly correlated with the level of antigen expressed, the fluorescent signal was calibrated with a known standard. This enabled the calculation of the level of antigen expression by determining the mean fluorescence, using flow cytometry analysis, and comparing the values found with those of the calibration curve.

WO 2013/146841 A1 discloses a medical image processor using bright field and fluorescent imaging of cells for identification of HER2 protein in the periphery of the cell membrane is related to the proliferation and malignant alteration of the cell. When there is cancer, overexpression of HER2 protein can be seen in the periphery of the cell membrane. Therefore, the number of fluorescent bright points showing the expression of the HER2 protein in the region of interest shows the amount of HER2 protein expressed in the periphery of the cell membrane for each cell in the region of interest and is a feature amount which quantitatively shows the degree of malignity of the cancer of the cells in the region of interest.

It is an object of the present invention to provide a method capable of more accurately quantifying a biological material expressed on the cell membrane in pathological samples while eliminating the influence of a degraded state, which is brought about in sample preparation, on fluorescence intensity. Solution to Problem

The inventors have accomplished the present invention based on the method according to the appended claims.

### Advantageous Effects of Invention

The use of the quantification method of the present invention makes it possible to increase the accuracy of the extraction and quantification of a target biological material expressed on the cell membrane and to improve the accuracy of pathological diagnosis, specifically, the accuracy of the decision for estimating the effect of molecular target drugs.

### Brief Description of Drawings

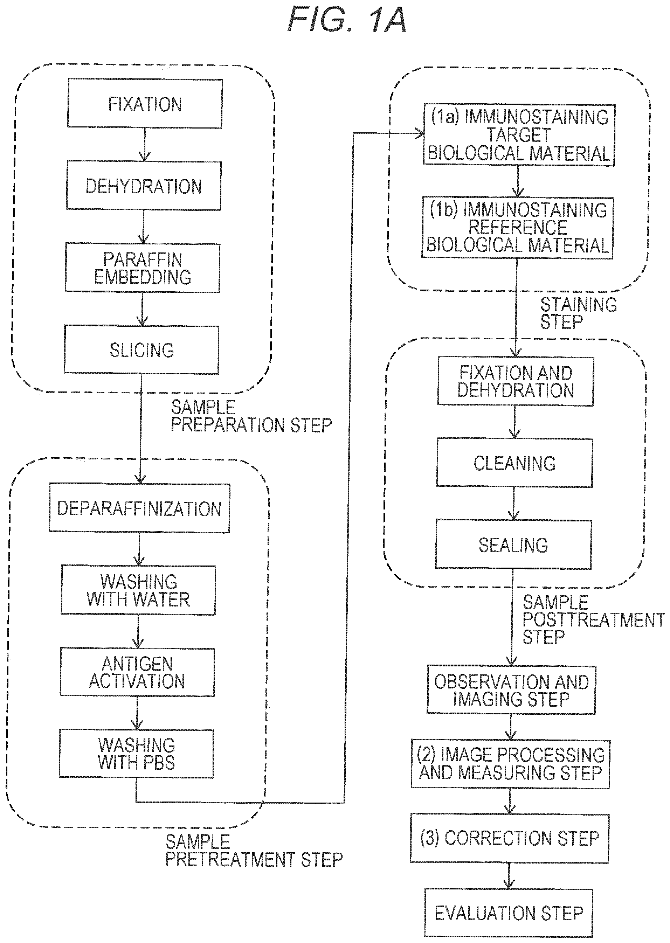

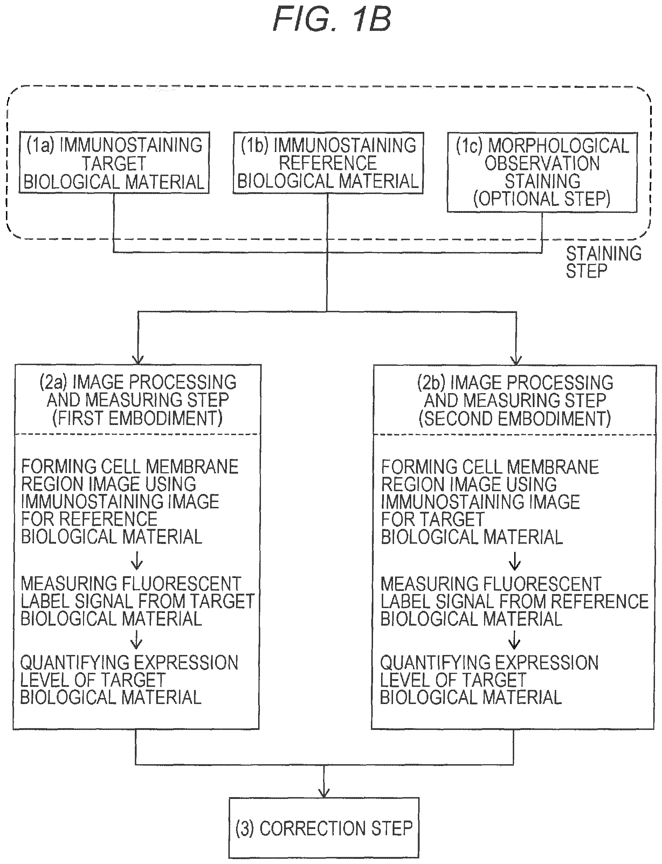

Fig. 1A is a flow chart showing an embodiment of the method according to the present invention for quantifying a biological material based on multiple immunostaining. Fig. 1A shows an example of the whole process while Fig. 1B shows only the excerpted steps related to claim 1.
Fig. 1B is a chart showing excerpted basic steps constituting the method of the present invention.
Fig. 2 is a first immunostaining image (shown in red in the color photograph) which is captured in Example 9 and in which a target biological material (HER2) is fluorescently labeled with Texas Red-containing melamine resin nanoparticles. The arrows indicate parts at which the target biological material is stained.
Fig. 3 is a second immunostaining image (shown in green in the color photograph) which is captured in Example 9 and in which reference biological materials (ATPase, cadherin, cytokeratin, and EpCAM) are fluorescently labeled with Alexa Fluor 647. The arrows indicate parts at which the reference biological materials are stained, and also indicate cell membrane regions.
Fig. 4 is an immunostaining image obtained by superimposing the first and second immunostaining images of Figs. 2 and 3. The arrows indicate parts at which the stained parts of the first and second immunostaining images overlap one another, in other words, indicate HER2 on cell membrane regions .

### Description of Embodiments

Target biological material and reference biological material

In the present invention, the target biological material as a first biological material refers to a biological material (specifically, a protein (antigen)) that is expressed on the cell membrane of a tissue section and is a target for immunostaining with a fluorescent label for quantification or detection mainly for pathological diagnosis.

In the present invention, the reference biological material as a second biological material refers to a biological material (specifically, a protein (antigen)) that is constantly expressed on the cell membrane of a tissue section, differs from the target biological material, and is a target for immunostaining with a fluorescent label for correction for increasing the accuracy of quantification or detection of the target biological material.

The target biological material is not limited and may be selected taking into account the use of the quantification method of the present invention, such as pathological diagnosis. Typical examples of the target biological material include biological materials that are expressed on cell membranes of various cancer tissues and useful as biomarkers, such as EGFR (HER1) (epidermal growth factor receptor), HER2 (human epidermal growth factor receptor), HER3, HER4, VEGFR (vascular endothelial growth factor receptor), IGFR (insulin-like growth factor receptor), HGFR (hepatocyte growth factor receptor), and other growth factor receptors; PD-1 (programmed cell death 1) and other immune system receptors; and other proteins. EGFR/HER is intended to include EGFR/HER1 (also called ErbB1) excessively expressed in colon cancer and other cancer tissues, EGFR2/HER2 (also called ErbB2 or neu) excessively expressed in breast cancer and other cancer tissues, EGFR3/HER3, and EGFR4/HER4. VEGFR is intended to include VEGFR-1 (also called Flt-1), the expression of which increases in the vascular endothelial cells of liver cancer, esophageal cancer, or other cancer tissues, VEGFR-2 (also called Flt-2 or KDR), and VEGFR-3 (also called Flt-4), the expression of which increases in lymphatic endothelial cells. For example, HER2 is preferred as the target biological material when the quantification method of the present invention is performed in pathological diagnosis of breast cancer.

In contrast to the target biological material, the reference biological material should be appropriately selected from biological materials that do not significantly change in expression amount even in a specific disease. In the present invention, preferred examples of the reference biological material may include membrane proteins such as ATPase, cadherin, cytokeratin, and EpCAM (epithelial cell adhesion/activating molecule, also called CD326, KSA, or TROP1).

Although there may be a single reference biological material, there are preferably two or more reference biological materials. When there are two or more reference biological materials, variations in the total expression level of them can be kept smaller, and the cell membrane image (cell membrane region) can be formed with a large number of fluorescent label molecules, so that the reference biological materials can be more stable.

### Immunostaining agents

In the present invention, a first immunostaining agent is a material for fluorescently labeling the target biological material by immunostaining and includes at least a fluorescent material and a probe capable of specifically binding to the target biological material.

In the present invention, a second immunostaining agent is a material for fluorescently labeling the reference biological material by immunostaining and includes at least a fluorescent material and a probe capable of specifically binding to the reference biological material.

### (Probe)

The probe (first probe) for the first immunostaining agent may be an antibody (IgG) capable of specifically recognizing a certain protein, which corresponds to the target biological material mentioned above, as an antigen and specifically binding to it. For example, when EGFR is the target biological material, an anti-EGFR antibody may be used as the first probe, and when HER2 is the target biological material, an anti-HER2 antibody may be used as the first probe.

The probe (second probe) for the second immunostaining agent may be an antibody (IgG) capable of specifically recognizing a certain protein, which corresponds to the reference biological material mentioned above, as an antigen and specifically binding to it. For example, when cadherin is the target biological material, an anti-cadherin antibody may be used as the second probe.

In view of the stability of quantification, the antibodies as the first and second probes are preferably monoclonal antibodies, although both antibodies may be polyclonal antibodies. Any kind of animals (immune animals) may be used to produce the antibodies. As conventional, such animals may be selected from, for example, mice, rats, guinea pigs, rabbits, goats, and sheep.

The antibody as the probe may also be an antibody fragment or an antibody derivative, rather than a natural full-length antibody, as long as it has the ability to specifically recognize a specific biological material (antigen) and to specifically bind to it. Therefore, as used herein, the term "antibody" is intended to include not only full-length antibodies but also antibody fragments such as Fab, F(ab)'2, Fv, and scFv and derivatives such as chimeric antibodies (such as humanized antibodies) and polyfunctional antibodies.

### (Fluorescent material)

The fluorescent material (first fluorescent material) for the first immunostaining agent and the fluorescent material (second fluorescent material) for the second immunostaining agent are not limited and may be selected from various known fluorescent materials used in the art. Typical examples of the fluorescent material include inorganic semiconductor nanoparticles (also called "quantum dots"), organic fluorescent dyes, and integrated materials thereof.

As used herein, the term "fluorescent material" refers to a material capable of emitting "fluorescence" and being bonded to the "probe," in which fluorescence occurs when electrons are excited upon absorption of the energy of an electromagnetic wave (X-ray, ultraviolet light, or visible light) applied at a specific wavelength and then fall back from the exited state to the ground state so that the resulting surplus energy is emitted as an electromagnetic wave. The term "fluorescence" has a wide meaning including fluorescence in a narrow sense, which has a short emission lifetime, and phosphorescence, which has a long emission lifetime and lasts even when the application of an electromagnetic wave for excitation is stopped.

### Inorganic semiconductor nanoparticles

Inorganic semiconductor nanoparticles may include a Group II-VI compound, a Group III-V compound, or a Group IV element-containing semiconductor, such as CdSe, CdS, CdTe, ZnSe, ZnS, ZnTe, InP, InN, InAs, InGaP, GaP, GaAs, Si, or Ge. Core/shell type inorganic semiconductor nanoparticles each including an inorganic semiconductor nanoparticle as a core and a shell formed on the outside of the core may also be used, such as CdSe/ZnS, CdS/ZnS, InP/ZnS, InGaP/ZnS, Si/SiO₂, Si/ZnS, Ge/GeO₂, and Ge/ZnS.

### Organic fluorescent dye

Examples of the organic fluorescent dye include rhodamine dye molecules, squarylium dye molecules, cyanine dye molecules, aromatic ring-containing dye molecules, oxazine dye molecules, carbopyronin dye molecules, and pyrromethene dye molecules. Other examples that may be used include Alexa Fluor (registered trademark) series of dye molecules (manufactured by Invitrogen), BODIPY (registered trademark) series of dye molecules (manufactured by Invitrogen), Cy (registered trademark) series of dye molecules (manufactured by GE Healthcare), DY (registered trademark) series of dye molecules (manufactured by Dyomics GmbH), HiLyte (registered trademark) series of dye molecules (manufactured by AnaSpec, Inc.), DyLight (registered trademark) series of dye molecules (manufactured by Thermo Scientific), ATTO (registered trademark) series of dye molecules (manufactured by ATTO-TEC GmbH), and MFP (registered trademark) series of dye molecules (manufactured by MoBiTec GmbH) . The nomenclature of generic names of such dye molecules is based on the principal structure (skeleton) or registered trademarks of the compounds, and those skilled in the art can appropriately understand the range, to which each fluorescent dye belongs, without undue trials and errors.

### Fluorophore integrated nanoparticles

Integrated fluorescent materials include "fluorophore integrated nanoparticles," which are nanosized particles each having a structure including an organic or inorganic material particle as a matrix and a plurality of fluorophore molecules contained in the matrix and/or adsorbed on the surface of the matrix. In this case, the matrix (e.g., a resin) and the fluorophore (e.g., an organic fluorescent dye) preferably have substituents or moieties with opposite charges, which electrostatically interact with each other.

Examples of the fluorophore to be contained in the fluorophore integrated nanoparticles include not only inorganic semiconductor nanoparticles and fluorescent dye molecules, as shown above, but also "long persistence fluorophores" composed of a base material such as Y₂O₃ or Zn₂SiO₄ and an activator such as Mn²⁺ or Eu³⁺.

The matrix used to form the fluorophore integrated nanoparticles may be an organic material, examples of which include melamine resins, urea resins, aniline resins, guanamine resins, phenolic resins, xylene resins, furan resins, and other resins generally classified as thermosetting resins; styrene resins, acrylic resins, acrylonitrile resins, AS resins (acrylonitrile-styrene copolymers), ASA resins (acrylonitrile-styrene-methyl acrylate copolymers), and other resins generally classified as thermoplastic resins; other resins such as polylactic acid; and polysaccharides, or may be an inorganic material such as silica (glass).

The fluorophore integrated nanoparticles can be produced by a known method (see, for example, JP 2013-57937 A) . More specifically, for example, fluorophore-containing silica particles each including silica as a matrix and a fluorophore contained therein can be produced by adding a solution of a fluorescent material such as inorganic semiconductor nanoparticles or an organic fluorescent dye and a silica precursor such as tetraethoxysilane dropwise to a solution of ethanol and ammonia to hydrolyze the silica precursor. On the other hand, fluorophore-containing resin particles each including a resin as a matrix and a fluorophore adsorbed on the surface of the resin particle or contained in the resin particle can be produced by previously preparing a solution of the resin or a dispersion of fine particles of the resin, adding a fluorescent material such as inorganic semiconductor nanoparticles or an organic fluorescent dye to the solution or dispersion, and stirring them.

Alternatively, a fluorescent dye may be added to a solution of raw materials for the resin, and then a polymerization reaction may be allowed to proceed to form fluorophore-containing resin particles. For example, a thermosetting resin such as a melamine resin may be used as the matrix resin. In this case, a reactive mixture containing a raw material for the resin (a monomer, an oligomer, or a prepolymer, such as methylolmelamine, a condensate of melamine and formaldehyde) and an organic fluorescent dye, and preferably further containing a surfactant and a polymerization reaction accelerator (such as an acid) may be heated to undergo an emulsion polymerization reaction, so that organic fluorescent dye-containing resin particles can be obtained. A thermoplastic resin such as a styrene-based copolymer may also be used as the matrix resin. In this case, a reactive mixture containing raw materials for the resin, an organic fluorescent dye (an organic fluorescent dye may be previously bonded to a raw material monomer for the resin via a covalent bond or the like, and the resulting monomer material may be used), and a polymerization initiator (such as benzoyl peroxide or azobisisobutyronitrile) may be heated to undergo a radical polymerization reaction or an ionic polymerization reaction, so that organic fluorescent dye-containing resin particles can be obtained.

The fluorophore integrated nanoparticles (specifically, fluorescent dye-containing resin particles obtained by the production method described above) may have any average particle size suitable for the immunostaining of pathological samples. In view of ease of detection of them as bright spots, they generally have an average particle size of 10 to 500 nm, preferably 50 to 200 nm. The coefficient of variation used to indicate variations in particle size is generally 20% or less, preferably from 5 to 15%. The fluorophore integrated nanoparticles satisfying these conditions can be produced under controlled conditions. For example, when the fluorophore integrated nanoparticles are produced by emulsion polymerization, the particle size can be controlled by the added amount of the surfactant. Generally, in this case, the particle size tends to decrease as the amount of the surfactant increases relative to the amount of the raw material for the matrix of the fluorophore integrated nanoparticles, whereas the particle size tends to increase as the amount of the surfactant decreases relative to the amount of the raw material.

The particle size of the fluorophore integrated nanoparticles can be determined as follows. A scanning electron microscope (SEM) is used to take an electron micrograph of fluorophore integrated nanoparticles. The cross-sectional areas of fluorophore integrated nanoparticles are measured in the electron micrograph. Assuming that the cross-sectional shape is a circle, the particle size can be calculated as the diameter of a circle corresponding to the cross-sectional area. The average particle size and coefficient of variation of a population of fluorophore integrated nanoparticles can be determined as follows. The particle sizes of a sufficient number (e.g., 1,000) of fluorophore integrated nanoparticles are calculated as described above. Subsequently, the average particle size is calculated as the arithmetic mean of the calculated particle sizes, and the coefficient of variation is calculated from the formula: 100 × the standard deviation of the particle sizes/the average particle size.

### Preferred fluorescent material

The first fluorescent material is preferably fluorescent dye-containing nanoparticles, and the second fluorescent material is preferably a fluorescent dye. This is because the fluorescent dye-containing nanoparticles for fluorescently labeling the target biological material can emit fluorescence with an intensity enough to show molecules of the target biological material one by one as bright spots and because the fluorescent dye for fluorescently labeling the reference biological material can emit fluorescence with an intensity reflecting the degraded state at the time of sample preparation so that the signal (the bright spot) from the target biological material can be properly corrected.

It will be understood that the embodiment using these first and second fluorescent materials is not intended to limit the present invention. For example, in another embodiment, inorganic semiconductor nanoparticles may be used as the first fluorescent material, and a fluorescent dye may be used as the second fluorescent material. A further embodiment may use fluorescent dye-containing nanoparticles in which the first and second fluorescent materials are capable of emitting fluorescence at different wavelengths. To immunostain two or more reference biological materials, it is not necessary to label them with different fluorescent materials capable of emitting fluorescence at different wavelengths, and a single common fluorescent material may be used as the second fluorescent material.

### (Features of immunostaining agents)

The first and second immunostaining agents are not limited and may be any of various known immunostaining agents capable of labeling the target and reference biological materials, respectively, and emitting signals that allow data useful for pathological diagnosis to be obtained. The immunostaining agent with such features for use may include a probe and a fluorescent material directly linked together by a covalent bond optionally with a linker molecule in between. However, in order to improve fluorescent labeling efficiency and thus to reduce the time going to lead to degradation of fluorescence, the immunostaining agent to be used is preferably a complex in which the probe and the fluorescent material are linked indirectly, in other words, the probe and the fluorescent material are linked through a non-covalent bond formed using an antigen-antibody reaction, an avidin-biotin reaction, or the like.

The fluorescent material in the first immunostaining agent for fluorescently labeling the target biological material is preferably fluorescent dye-containing nanoparticles as mentioned above. An example of the first immunostaining agent having a probe and a fluorescent material linked indirectly may be a complex including three molecules linked according to the following formula: [an antibody (a primary antibody as a probe) to the target biological material]...[an antibody (secondary antibody) to the primary antibody]-[biotin]/[avidin]-[a fluorescent material (fluorescent dye-containing nanoparticle)], wherein "..." represents bonding by an antigen-antibody reaction, "-" represents covalent bonding optionally with a linker molecule in between, and "/" represents bonding by an avidin-biotin reaction.

On the other hand, the fluorescent material in the second immunostaining agent for fluorescently labeling the reference biological material is preferably a fluorescent dye as mentioned above. An example of the second immunostaining agent having a probe and a fluorescent material linked indirectly may be a complex including two molecules linked according to the following formula: [an antibody (a primary antibody as a probe) to the reference biological material]...[an antibody (secondary antibody) to the primary antibody]-[a fluorescent material (fluorescent dye)], wherein "..." represents bonding by an antigen-antibody reaction, and "-" represents covalent bonding optionally with a linker molecule in between.

In an embodiment using a combination of these complexes, the secondary antibody for binding to the primary antibody to the target biological material is preferably of a different type from that of the secondary antibody for binding to the primary antibody to the reference biological material so that unintentional binding can be avoided. For example, a mouse IgG may be used as the primary antibody to the target biological material and an anti-mouse IgG antibody may be used as the secondary antibody thereto, while a rabbit IgG may be used as the primary antibody to the reference biological material and an anti-rabbit IgG antibody may be used as the secondary antibody thereto. The primary and secondary antibodies may be prepared by conventional methods or used from commercially available sources.

The secondary antibody-biotin complex (biotin-modified secondary antibody) can be prepared, for example, using commercially available biotin labeling reagents (kit) based on a known technique capable of binding biotin to the desired antibody (protein). A biotin-modified secondary antibody having biotin bound to the desired antibody in advance may also be used if commercially available.

The fluorescent material-avidin complex (avidin-modified fluorescent material) can also be prepared, for example, using commercially available avidin labeling reagents (kit) based on a known technique capable of binding avidin to the fluorescent material. In this case, avidin may be replaced with a modified version of avidin, such as streptavidin or NeutrAvidin, which has higher binding strength to biotin than avidin.

Exemplary methods for preparing the fluorescent material-avidin complex may be as follows. When the fluorescent material is fluorophore integrated nanoparticles containing a resin matrix, the functional group of the resin and the functional group of avidin (protein) may be linked with a linker molecule, such as PEG, optionally having functional groups at both ends of the molecule. For example, when a melamine resin is used, the functional group of the melamine resin, such as an amino group may be used. In the case of an acrylic or styrene resin, a monomer having a functional group (e.g., an epoxy group) on the side chain may be used to form a copolymer, in which the functional group itself may be used or another functional group formed by conversion of the functional group (e.g., an amino group formed by reaction with ammonia water) may be used, and any other functional group may also be introduced using these functional groups. When the fluorescent material is inorganic semiconductor nanoparticles or fluorophore integrated nanoparticles containing a silica matrix, the desired functional group can be introduced by surface modification with a silane coupling agent (for example, an amino group can be introduced using aminopropyltrimethoxysilane). On the other hand, a thiol group can be introduced into avidin by allowing the amino group of avidin to react with, for example, N-succinimidyl S-acetylthioacetate (SATA). The fluorescent material having the amino group and avidin with the introduced thiol group can be linked using N-hydroxysuccinimide (NHS) ester reactive with the amino group and a cross-linker reagent having a polyethylene glycol (PEG) chain and maleimide groups (reactive with the thiol group) at both ends thereof.

The secondary antibody-fluorescent dye complex (fluorescently labeled secondary antibody) can be prepared, for example, using commercially available fluorescent labeling reagents (kit) based on a known technique capable of binding the desired fluorescent dye to the desired antibody (protein) . A fluorescently labeled secondary antibody having the desired fluorescent dye bound to the desired antibody in advance may also be used if commercially available.

### Method for quantifying target biological material (see the drawings)

The method according to the present invention for quantifying a target biological material includes at least the steps defined in appended claim 1.

The first and second immunostaining steps may be performed in any desired order or at the same time.

The method according to the present invention for quantifying a target biological material may generally include additional steps other than the above steps, such as a sample pretreatment step and a sample posttreatment step. Specifically, these steps may be performed, for example, by the following procedures.

### (Sample pretreatment step)

Pathological samples are often stored in the form of paraffin-embedded sections of cells, tissues, or organs. In order to immunostain such sections, the sections are generally subjected to deparaffinization and antigen activation before the immunostaining.

Deparaffinization may include, for example, immersing the pathological section in xylene to remove paraffin, then immersing the pathological section in ethanol to remove xylene, and immersing the pathological section in water to remove ethanol. In general, these three steps may be performed at room temperature . Each immersion time may be about 3 to about 30 minutes, and if necessary, each treatment liquid may be replaced during the immersion.

The antigen activation may include, for example, immersing, after the deparaffinization, the pathological sample in an activation solution (such as a 0.01 M citric acid buffer, a 1 mM EDTA aqueous solution, a 5% urea aqueous solution, or a 0.1 M Tris-hydrochloric acid buffer), heating the immersed sample, and then cleaning the pathological sample by immersing it in PBS. The heating in the activation solution may be performed at a temperature of 50 to 130°C for a time period of 5 to 30 minutes . The immersion in PBS may be generally performed at room temperature for about 3 to about 30 minutes, and if necessary, PBS may be replaced with new one during the immersion.

### (Staining step)

### (1a) First immunostaining step (immunostaining the target biological material)

The first immunostaining step for immunostaining the target biological material includes immunostaining a pathological sample using the first immunostaining agent described above. When the first immunostaining agent has the probe and the fluorescent material linked directly, the pathological sample after the activation step may be immersed in a solvent containing the first immunostaining agent. Alternatively, when the first immunostaining agent is a complex having the probe and the fluorescent material linked indirectly, the pathological sample may be sequentially immersed in a plurality of reagents for forming the complex.

For example, when the first immunostaining agent is a complex of the formula: [a primary antibody (probe)] ... [a secondary antibody]-[biotin]/[avidin]-[fluorescent dye-containing nanoparticle (fluorescent material)], the staining step may include first immersing the pathological sample in a primary antibody (probe) solution (also referred to as "reagent I" in the description) (primary reaction treatment); then immersing the pathological sample in a secondary antibody-biotin complex solution (also referred to as "reagent II" in the description) (secondary reaction treatment); and finally immersing the pathological sample in an avidin-fluorescent dye-containing nanoparticles solution (also referred to as "reagent III" in the description)

### (fluorescent labeling treatment).

The conditions for the first immunostaining step, such as the temperature and time of the immersion of the pathological sample in the given solution (reagent) in each of the primary and secondary reaction treatments and the fluorescent labeling treatment, may be appropriately controlled according to conventional immunostaining methods to produce suitable signals.

### (1b) Second immunostaining step (immunostaining the reference biological material)

The second immunostaining step for immunostaining the reference biological material includes immunostaining the pathological sample using the second immunostaining agent described above. When the second immunostaining agent has the probe and the fluorescent material linked directly, the pathological sample after the activation step may be immersed in a solvent containing the second immunostaining agent. Alternatively, when the second immunostaining agent is a complex having the probe and the fluorescent material linked indirectly, the pathological sample may be sequentially immersed in a plurality of reagents for forming the complex.

For example, when the second immunostaining agent is a complex of the formula: [a primary antibody (probe)] ... [a secondary antibody]-[fluorescent dye (fluorescent material)], the staining step may include first immersing the pathological sample in a primary antibody (probe) solution (also referred to as "reagent i" in the description) (primary reaction treatment); and then immersing the pathological sample in a secondary antibody-fluorescent dye complex solution (also referred to as "reagent ii" in the description) (secondary reaction-fluorescent labeling treatment).

When there are two or more reference biological materials, the reagent i is preferably designed to contain two or more antibodies to the reference biological materials, respectively, so that all the reference biological materials can be immunostained at a time.

The conditions for the second immunostaining step, such as the temperature and time of the immersion of the pathological sample in the given solution (reagent) in each of the primary reaction treatment and the secondary reaction-fluorescent labeling treatment, may be appropriately controlled according to conventional immunostaining methods to produce suitable signals.

### (1c) Optional step

If necessary, the method according to the present invention may include a morphological observation staining step so that the morphology of cells, tissues, or organs can be observed in the bright field. The morphological observation staining step may be performed according to a conventional method. For the morphological observation of tissue samples, eosin staining is standardly used, in which cytoplasm, interstitium, various fibers, erythrocytes, and keratinocytes are stained in red to dark red. Hematoxylin staining is also standardly used, in which cell nuclei, calciferous parts, cartilage tissues, bacteria, and mucosa are stained in blue-purple to light blue (a method of performing the two staining techniques is known as hematoxylin-eosin staining (HE staining)). If included, the morphological observation staining step may be performed before or after the immunostaining step.

### (Sample posttreatment step)

After the first and second immunostaining steps, the pathological sample is preferably subjected to treatments for making the pathological sample suitable for observation, such as fixation and dehydration, cleaning, and sealing.

The fixation and dehydration treatment may include immersing the pathological sample in a fixing liquid (a cross-linking agent such as formalin, paraformaldehyde, glutaraldehyde, acetone, ethanol, or methanol). The cleaning may include immersing the pathological sample in a cleaning liquid (such as xylene) after the fixation and dehydration treatment. The sealing treatment may include immersing the pathological sample in a sealing liquid after the cleaning treatment. The conditions for these treatments, such as the temperature and time of the immersion of the pathological sample in the given treatment liquid, may be appropriately controlled according to conventional immunostaining methods to produce suitable signals.

### (Observation and imaging step)

The observation and imaging step may include obtaining a staining image (first immunostaining image) produced by the first immunostaining step and obtaining a staining image (second immunostaining image) produced by the second immunostaining step. Specifically, in the same field of view of a microscope at a desired magnification, light for exciting the first fluorescent material, which is used to fluorescently label the target biological material in the first immunostaining step, and light for exciting the second fluorescent material, which is used to fluorescently label the reference biological material in the second immunostaining step, are applied to the pathological sample, respectively, and the immunostaining images produced by the fluorescence emitted from these fluorescent materials are observed and captured. In the observation step, the exciting light preferably has a wavelength of 550 to 630 nm in order to prevent tissue sections from producing too high self-fluorescence, although the wavelength of the exciting light is not limited as long as the fluorescence from the fluorescent materials for immunostaining agents is detectable relative to the self-fluorescence from the tissue sections. These exciting light beams may be applied using, for example, a laser light source in a fluorescence microscope and optionally an exciting light filter capable of selectively transmitting specific wavelengths. When the wavelength of light for exciting the first fluorescent material differs from that of light for exciting the second fluorescent material, exciting light filters may be switched so that two wavelengths of exciting light can be applied from a single laser light source. The immunostaining images can be captured by, for example, a digital camera in a fluorescence microscope. In the process of capturing the immunostaining images, if necessary, a fluorescence filter capable of selectively transmitting specific wavelengths may be used so that the immunostaining images can be captured so as to include only the desired fluorescence and to exclude undesired fluorescence, exciting light as a noise source, and other types of light. The wavelengths of fluorescence from the first and second fluorescent materials need to be distinguishably different. To meet this requirement, fluorescence filters may be switched while the respective fluorescent immunostaining images are captured.

### (Image processing and measuring step)

The image processing and measuring step may include performing image processing on each of the first immunostaining image captured for the target biological material and the second immunostaining image captured for the reference biological material; measuring the fluorescent label signal corresponding to the target biological material and the fluorescent label signal corresponding to the reference biological material based on the results of the image processing; and identifying the position of the fluorescent label signal corresponding to the target biological material in a cell membrane region.

The fluorescent label signal may be handled as the intensity of fluorescence (brightness) or as the number of bright spots of fluorescence. When fluorescent dye-containing nanoparticles are used as the fluorescent material for the target biological material, the signals are preferably handled as bright spots. When a fluorescent dye is used as the fluorescent material for the reference biological material, the signal is preferably handled as the intensity of fluorescence.

The software used for the image processing may be, for example, ImageJ (open source). The use of such image processing software makes it possible to semi-automatically and quickly perform the following processing: extracting bright spots with a given wavelength (color) from the immunostaining image and calculating the sum total of the degrees of brightness; and measuring the number of bright spots with at least a certain level of brightness, specifically processing for the first and second embodiments described below.

In the present invention, the use of the first and second staining images makes it possible to measure the fluorescent label signals and at the same time to specify and extract the fluorescent label signal corresponding to the target biological material in a certain cell membrane region of the staining image (specifically, on the cell membrane). Particularly when fluorescent dye-containing nanoparticles are used as the fluorescent material for the target biological material and a fluorescent dye is used as the fluorescent material for the reference biological material, preferred embodiments include the first and second embodiments described below.

### (2a) First Embodiment

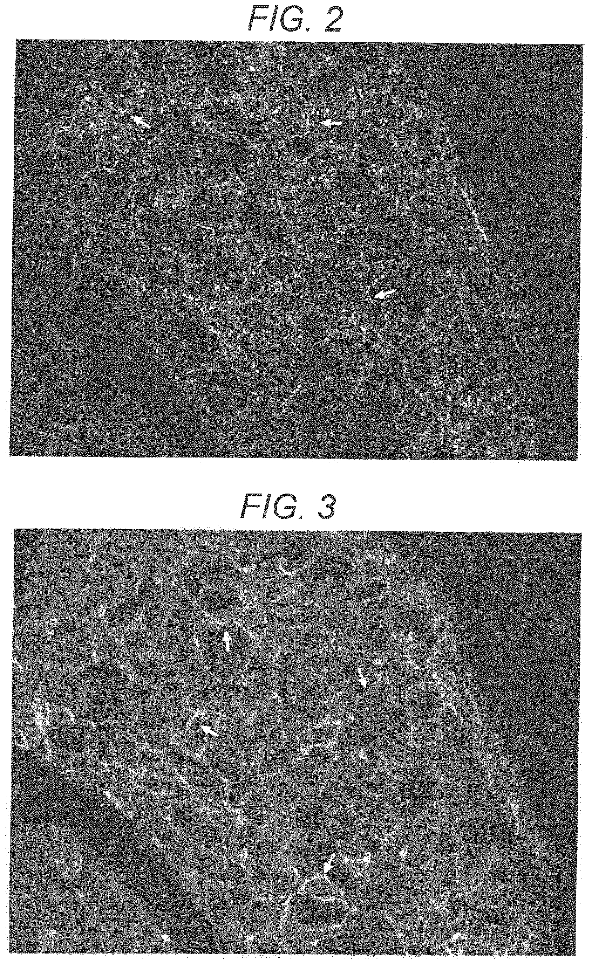

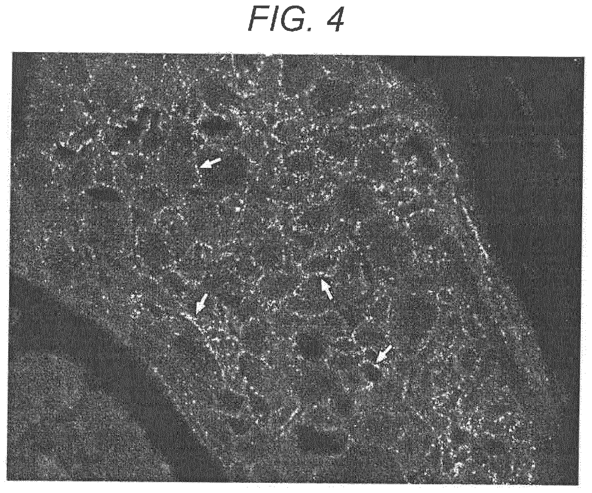

The first embodiment includes the steps of:
(2a-1) extracting bright spots of the fluorescent dye from the immunostaining image (see Fig. 3) for the reference biological material to form an cell membrane region image and measuring, as the fluorescent label signal from the reference biological material, the sum total of the degrees of brightness of the bright spots in the cell membrane region;
(2a-2) superimposing (see Fig. 4) the immunostaining image (see Fig. 2) for the target biological material on the cell membrane region image and measuring, as the fluorescent label signal from the target biological material, the number of bright spots of the fluorescent dye-containing nanoparticles inside the cell membrane region; and
(2a-3) dividing the number of bright spots in the cell membrane region by the sum total of the degrees of brightness of the fluorescent dye to obtain a corrected measured value as an index value for quantifying the expression level of the target biological material.

In the first embodiment, therefore, the immunostaining image for the reference biological material is used to correct the fluorescent label signal from the target biological material, and a cell membrane region image is formed and then used for the extraction of only the fluorescent label signal (bright spot) from the target biological material in the cell membrane region. Therefore, additional staining for detecting the cell membrane (such as DAB staining observable in the bright field) is unnecessary in the present invention.

### (2b) Second Embodiment

The second embodiment includes the steps of:
(2b-1) extracting bright spots of the fluorescent dye-containing nanoparticles from the immunostaining image (see Fig. 2) for the target biological material according to a bright spot extraction program to form a cell membrane region image and measuring, as the fluorescent label signal from the target biological material, the number of the bright spots in the cell membrane region;
(2b-2) superimposing the immunostaining image (see Fig. 3) for the reference biological material on the cell membrane region and measuring, as the fluorescent label signal from the reference biological material, the sum total of the degrees of brightness of the fluorescent dye inside the cell membrane region; and
(2b-3) dividing the number of the bright spots in the cell membrane region by the sum total of the degrees of brightness of the fluorescent dye to obtain a corrected measured value as an index value for quantifying the expression level of the target biological material.

The bright spot extraction problem in the step (2b-1) may be based on a method capable of properly extracting bright spots even when there are variations in brightness, such as the top-hat method.

In the second embodiment with such features, the use of a suitable bright spot extraction problem makes it possible to form a cell membrane region image even from only the immunostaining image for the target biological material and to extract only the fluorescent label signals (bright spots) from the target biological material in the cell membrane region. The immunostaining image for the reference biological material may be used only to calculate the sum total of the degrees of brightness for the correction of the fluorescent label signal (bright spots) from the target biological material.

### (Correction step)

The correction step includes correcting the measured value of the fluorescent label signal corresponding to the target biological material in the cell membrane region using the measured value of the fluorescent label signal corresponding to the reference biological material, for the whole measurement region or for each cell, so that an index for quantifying the expression level is obtained. The correction may be made by any method such as a method of subjecting each measured signal value to conversion, transformation, or comparison. Specifically, a preferred correction method includes using the number of bright spots of fluorescent dye-containing nanoparticles as the fluorescent label signal corresponding to the target biological material, using the sum total of the degrees of brightness of the fluorescent dye as the fluorescent label signal corresponding to the reference biological material, and making a correction by dividing the former by the latter.

In this regard, such a correction may be made for the whole of the regions measured for the first and second immunostaining images (e.g., the whole field of view or the whole of any other regions of interest for measurement) or may be made for each cell in the region.

### (Evaluation step)

The evaluation step may include evaluating the sample by quantifying the target biological material in the cell membrane region based on the index obtained in the correction step, namely, based on the value obtained by correcting the measured value of the fluorescent label signal corresponding to the target biological material in the cell membrane region, to obtain useful information for pathological diagnosis.

The method for evaluating the sample may be similar to pathological diagnosis based on conventional immunohistochemical (IHC) staining. For example, for pathological diagnosis of breast cancer, a method of evaluating the expression level of HER2 protein in pathological samples is widely used to evaluate the therapeutic efficacy of Herceptin, a molecular target drug specifically targeting HER2 protein ("HER2 Test Guide for Proper Case Selection for Herceptin," September, 2009, prepared by the Trastuzumab Pathology Committee, Third Edition). This method includes immunostaining HER2 by DAB staining or the like according to the given protocol, determining a score (3+, 2+, 1+, or 0) from the results of the staining pattern (the rate of positively stained cancer cells of the cell membrane in the sample), and determining whether it is positive or negative (3+ = positive, 2+ = equivocal, 1+ or 0 = negative) . Subsequently, whether or not the subject is to be treated with Herceptin is determined using this result in combination with the result of evaluation of overexpression of HER2 gene by FISH method, which is preformed separately. In such pathological diagnosis, the corrected value obtained by the method of the present invention to quantify the expression level of HER2 on the cell membrane is statistically compared with results on Herceptin response rate, so that scores and evaluations similar to those for conventional pathological diagnosis can be set to enable effective pathological diagnosis.

### Kit

Another aspect of the present invention provides a kit for carrying out the method according to the present invention for quantifying a target biological material. This kit is as defined in appended claim 10.

Specifically, for example, the kit preferably includes components capable of forming, as an immunostaining agent for a target biological material, a complex of the formula: [a primary antibody (probe)]...[a secondary antibody]-[biotin]/[avidin]-[fluorescent dye-containing nanoparticle (fluorescent material)] shown above, in which the components include a primary antibody; a secondary antibody-biotin complex or a secondary antibody, biotin, and a reaction reagent for forming the secondary antibody-biotin complex; an avidin-fluorescent dye-containing nanoparticle complex or avidin, fluorescent dye-containing nanoparticles, and a reaction reagent for forming the avidin-fluorescent dye-containing nanoparticle complex. The kit also preferably includes components capable of forming, as an immunostaining agent for a reference biological material, a complex of the formula: [a primary antibody (probe)]...[a secondary antibody]-[fluorescent dye (fluorescent material)] shown above, in which the components include a primary antibody (when there are two or more reference biological materials, antibodies for these reference biological materials, respectively); and a secondary antibody-fluorescent dye complex or a secondary antibody, a fluorescent dye, and a reaction reagent for forming the secondary antibody-fluorescent dye complex.

If necessary, the kit may further include other necessary reagents and components for immunostaining pathological samples, an instruction sheet that describes how to carry out the method according to the present invention for quantifying a target biological material, and other optional components.

### Examples

### [Reagent II: Preparation of biotin-modified anti-rabbit IgG antibody]

Fifty µg of an anti-rabbit IgG antibody for use as a secondary antibody was dissolved in a 50 mM Tris solution. DTT (dithiothreitol) was added to the solution to reach a final concentration of 3 mM and allowed to react at 37 °C for 30 minutes with mixing. Subsequently, the DTT-reduced secondary antibody was purified by allowing the reaction solution to pass through desalting columns, Zeba Desalt Spin Columns (Cat. #89882, Thermo Scientific). An antibody solution was prepared by dissolving 200 µL part of the whole purified antibody in a 50 mM Tris solution. On the other hand, a linker reagent Maleimide-PEG2-Biotin (Catalog number: 21901, Thermo Scientific) was adjusted to 0.4 mM with DMSO. To the antibody solution was added 8.5 µL of the linker reagent solution and allowed to react at 37°C for 30 minutes with mixing, so that biotin was bound to the anti-rabbit IgG antibody via the PEG chain. The reaction solution was purified by being allowed to pass through a desalting column. The concentration of the protein (biotin-modified secondary antibody) in the desalted reaction solution was calculated through measuring the absorbance of the reaction solution at a wavelength of 300 nm with a spectrophotometer (F-7000 manufactured by Hitachi, Ltd.) . The concentration of the biotin-modified secondary antibody was adjusted to 250 µg/mL with a 50 mM Tris solution. The resulting solution was used as a biotin-modified secondary antibody (reagent II) solution.

### [Reagent III: Preparation of streptavidin-modified Texas Red dye-containing melamine resin nanoparticles]

### (1) Preparation of Texas Red dye-containing melamine resin nanoparticles

After 2.5 mg of Texas Red dye molecule Sulforhodamine 101 (Sigma-Aldrich) was dissolved in 22.5 mL of pure water, the solution was stirred for 20 minutes with a hot stirred while its temperature was kept at 70°C. After the stirring, 1.5 g of a melamine resin NIKALAC MX-035 (NIPPON CARBIDE INDUSTRIES CO., INC.) was added to the solution and then heated with stirring for 5 minutes under the same conditions. After the stirring, 100 µL of formic acid was added to the solution and then stirred for 20 minutes while the temperature of the solution was kept at 60°C. The solution was then allowed to cool to room temperature. After the cooling, the solution was dispensed into a plurality of centrifuge tubes and then centrifuged at 12,000 rpm for 20 minutes, so that Texas Red dye-containing melamine resin nanoparticles (hereinafter referred to as particles X), which were contained as a mixture in the solution, were precipitated. After the supernatant was removed, the precipitated particles X were washed with ethanol and water.

### (2) Preparation of maleimide group-modified, Texas Red dye-containing, melamine resin nanoparticles

After the washing, 0.1 mg of the particles X were dispersed in 1.5 mL of ethanol, to which 2 µL of aminopropyltrimethoxysilane LS-3150 (Shin-Etsu Chemical Co., Ltd.) was added. The mixture was subjected to surface amination by reaction at room temperature for 8 hours with stirring. The concentration of the surface-aminated particles X was adjusted to 3 nM with 2 mM EDTA (ethylenediamine tetraacetic acid)-containing PBS. A linker reagent SM(PEG)12 (Catalog number: 22112, Thermo Scientific) was added to the solution to reach a final concentration of 10 mM, mixed, and allowed to react at room temperature for 1 hour with stirring. The reaction liquid was centrifuged at 10,000 G for 20 minutes. After the supernatant was removed, the precipitate was dispersed by addition of 2 mM EDTA-containing PBS and then centrifuged again under the same conditions. After the same washing procedure was performed three times, PEG-chain-surface-modified particles X with terminal maleimide groups were obtained.

### (3) Preparation of thiol group-modified streptavidin

On the other hand, thiol group-modified streptavidin was prepared as described below. First, 70 µL of an aqueous solution of 64 mg/mL N-succinimidyl S-acetylthioacetate (SATA) (manufactured by Pirce) was added to 40 µL of an aqueous solution of 1 mg/mL streptavidin (Wako Pure Chemical Industries, Ltd.) and then allowed to react at room temperature for 1 hour, so that a protected thiol group (-NH-CO-CH₂-S-CO-CH₃) was introduced at the amino group of streptavidin. Subsequently, a free thiol group (-SH) was produced from the protected thiol group by a hydroxylamine treatment, so that the introduction of a thiol group (-SH) into streptavidin was completed. The resulting solution was desalted by being allowed to pass through gel filtration columns (Zaba Spin Desalting Columns, Funakoshi Co., Ltd.), so that thiol group-modified streptavidin was obtained.

### (4) Preparation of streptavidin-modified, Texas Red dye-containing, melamine resin nanoparticles

The PEG-chain-surface-modified particles X (Texas Red dye-containing melamine resin nanoparticles) with terminal maleimide groups prepared in section (2) and the thiol group-modified streptavidin prepared in section (3) were mixed in 2 mM EDTA-containing PBS and allowed to react for 1 hour, so that streptavidin was bound to the particles X via the PEG chain. The reaction was quenched by adding 10 mM mercaptoethanol to the reaction liquid. After the resulting solution was concentrated using a centrifuge filter, the unreacted material was removed using a gel filtration column for purification, so that streptavidin-modified particles X (reagent III) were obtained.

### [Example 1]

### (E1) Sample preparation step and sample pretreatment step

A cultured breast cancer cell line CRL1500 was fixed with 4% paraformaldehyde and then subjected to dehydration, embedding, and sectioning according to conventional methods, so that samples were obtained. Three samples were prepared for each of a 1 hour-fixation treatment group, a 12 hour-fixation treatment group, and a 96 hour-fixation treatment group.

The prepared samples were subjected to deparaffinization and then washing for replacement with water. The washed tissue array slides were autoclaved in a 10 mM citric acid buffer solution (pH 6.0) at 121°C for 15 minutes so that antigen activation was performed. After the activation, the tissue array slides were washed with PBS. The washed tissue array slides were subjected to a blocking treatment with 1% BSA-containing PBS for 1 hour.

### (E2) Immunostaining step

### (E2-1) Common primary reaction treatment for first immunostaining and second immunostaining

A primary reaction treatment for first immunostaining for the target biological material HER2 and a primary reaction treatment for second immunostaining for a reference biological material cadherin were performed at a time using a reaction treatment liquid prepared by dissolving antibodies for both of the primary reaction treatments. Specifically, 1% BSA-containing PBS was used to form a primary reaction treatment liquid containing 0.05 nM of an anti-HER2 rabbit monoclonal antibody 4B5 (Ventana) (reagent I) and a 1/200 dilution of an anti-pan cadherin antibody CH-19 (ab6528, Abcam). The samples prepared in the step (1) were allowed to react at 4°C overnight by being immersed in the primary reaction treatment liquid.

### (E2-2) Secondary reaction treatment for first immunostaining

The solution of the reagent II (biotin-modified anti-rabbit IgG antibody) prepared in the preparation example was further diluted to 6 µg/mL with 1% BSA-containing PBS to form a secondary reaction treatment liquid. After the primary reaction treatment, the samples were washed with PBS and then allowed to react at room temperature for 30 minutes by being immersed in the secondary reaction treatment liquid.

### (E2-3) Fluorescent labeling treatment for first immunostaining

The reagent III (streptavidin-modified, Texas Red dye-containing, melamine resin particles) prepared in the preparation example was diluted to 0.02 nM with 1% BSA-containing PBS to form a fluorescent labeling reaction treatment liquid. After the secondary reaction treatment, the samples were allowed to react at room temperature for 3 hours by being immersed in the fluorescent labeling treatment liquid under neutral pH conditions (pH 6.9 to 7.4).

### (E2-4) Secondary reaction and fluorescent labeling treatment for second immunostaining

Alexa Fluor 647-labeled anti-mouse IgG antibody (A21236, Life Technologies) was diluted to 1/200 with 1% BSA-containing PBS to form a secondary reaction treatment liquid. After the primary reaction treatment, the samples were washed with PBS and then allowed to react at room temperature for 30 minutes by being immersed in the secondary reaction treatment liquid.

### (E2-5) Sample posttreatment step

After the first immunostaining and the second immunostaining, the samples were subjected to fixation and dehydration, in which immersion in pure ethanol for 5 minutes was performed four times. Subsequently, the samples were subjected to a cleaning treatment, in which immersion in xylene for 5 minutes was performed four times. Finally, the samples were subjected to a sealing treatment including putting a sealant Entellan New (Merck) on the samples and then covering each sample with a cover glass, so that samples for use in observation were obtained.

### (E3) Evaluation step

### (E3-1) Observation and imaging step

In this step, a fluorescence microscope BX-53 (Olympus Corporation) was used for the irradiation with exciting light and the observation of emitted fluorescence, and a microscope digital camera DP73 (Olympus Corporation) attached to the fluorescence microscope was used to capture immunostaining images (at a magnification of × 400).

First, the sample was irradiated with exciting light for causing the Texas Red dye, which was used for the fluorescent labeling of the target biological material (HER2), to emit fluorescence, and the immunostaining image in that state was captured (the first immunostaining image) . In this process, the wavelength of the exciting light was set at 575 to 600 nm using an exciting light optical filter in the fluorescence microscope, and the wavelength of the fluorescence observed was set at 612 to 692 nm using a fluorescence optical filter. The observation with the fluorescence microscope and the intensity of the exciting light during the image capturing were such that an irradiation energy of 900 W/cm² was achieved at or near the center of the field of view. The exposure time during the image capturing was adjusted in the range where the brightness of the image was not saturated, and, for example, set at 4, 000 µsec.

Subsequently, the exciting light optical filter and the fluorescence optical filter were switched to other filters. The sample was irradiated with exciting light for causing Alexa Fluor 647, which was used for the fluorescent labeling of the reference biological material (cadherin), to emit fluorescence, and the immunostaining image in that state was captured (the second immunostaining image). In this process, the wavelength of the exciting light was set at 620 to 650 nm using an exciting light optical filter in the fluorescence microscope, and the wavelength of the fluorescence observed was set at 660 to 700 nm using a fluorescence optical filter. The observation with the fluorescence microscope and the intensity of the exciting light during the image capturing were such that an irradiation energy of 400 W/cm² was achieved at or near the center of the field of view. The exposure time during the image capturing was adjusted in the range where the brightness of the image was not saturated, and, for example, set at 800 msec.

After the first and second immunostaining images were captured in the same field of view, the same procedure was repeated in different fields of view. In total, the first and second immunostaining images were captured in five fields of view (first to fifth fields of view) for each sample.

### (E3-2) Image processing and measuring step

In this step, image processing was performed using image processing software ImageJ (open source).

First, using the second immunostaining image for cadherin, the reference biological material, regions in which the brightness of the fluorescence emitted by the fluorescent material was at least a predetermined value were extracted as the "bright spot measurement regions" corresponding to the target biological material-expressing cell membrane. In addition, the sum total (Y) of the intensities of the fluorescence defining the bright spot measurement regions was calculated.

Subsequently, the first immunostaining image for HER2, the target biological material, was superimposed on the second immunostaining image from which the bright spot measurement regions were extracted, and the number (X) of bright spots with at least a predetermined degree of brightness was measured among the bright spots positioned in the regions and indicating Texas Red dye-containing melamine resin particles with which HER2 was fluorescently labeled.

### (E3-3) Correction step

For correction, a value was obtained by dividing the X value by the Y value. After such a value was calculated for each of the first to fifth fields of view, the average of the resulting values was calculated as the "corrected HER2 quantification value" for use in the evaluation of the HER2 expression level.

A coefficient of variation CV (= 100 × the standard deviation of the corrected HER2 quantification values/the average of the corrected HER2 quantification values) was calculated from the corrected HER2 quantification values for 9 samples in total in the 1 hour-fixation treatment group (the number of samples n = 3), the 12 hour-fixation treatment group (n = 3), and the 96 hour-fixation treatment group (n = 3).

### [Example 2]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to SK-BR3.

### [Example 3]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to NDA-MB453.

### [Example 4]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to NDA-MB175.

### [Example 5]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to COLO201.

### [Example 6]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to Hela.

### [Example 7]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to MCF-7.

### [Example 8]

The sample preparation step, the immunostaining step, the evaluation step, and the calculation of the coefficient of variation of the corrected HER2 quantification value were performed by the same procedure as in Example 1, except that the breast cancer cell line used in the sample preparation step was changed from CRL1500 to NDA-MB231.

### [Example 9]

The number of reference biological materials was changed from one (cadherin) to four (ATPase, cadherin, cytokeratin, and EpCAM). Specifically, the primary reaction treatment liquid for the first immunostaining and the second immunostaining used in the immunostaining step contained 0.05 nM of an anti-HER2 rabbit monoclonal antibody 4B5 (Ventana) (reagent I) and 1/200 dilutions of an anti-pan cadherin antibody CH-19 (ab6528, Abcam), an anti-alpha 1 sodium potassium ATPase antibody 464.6-plasma membrane marker (ab7671, Abcam), an anti-pan cytokeratin antibody PCK-26 (ab6401, Abcam), and an anti-EpCAM antibody VU1D9 (Abcam), respectively. Except for the above, the sample preparation step, the immunostaining step, and the evaluation step were performed by the same procedure as in Example 1.

### [Example 10]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to SK-BR3.

### [Example 11]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to NDA-MB453.

### [Example 12]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to NDA-MB175.

### [Example 13]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to COLO201.

### [Example 14]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to Hela.

### [Example 15]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to MCF-7.

### [Example 16]

The sample preparation step, the immunostaining step, the evaluation step, and the calculation of the coefficient of variation of the corrected HER2 quantification value were performed by the same procedure as in Example 9, except that the breast cancer cell line used in the sample preparation step was changed from CRL1500 to NDA-MB231.

### [Example 17]

The sample preparation step, the immunostaining step, and the calculation of the coefficient of variation of the corrected HER2 quantification value were performed by the same procedure as in Example 1, except that HE staining was further performed after the immunostaining step. In the evaluation step of this example, the step of capturing an HE staining image in the bright field was performed in addition to the step of capturing the immunostaining images while irradiating the sample with exciting light for causing the dyes, which were used for the fluorescent labeling of the target and reference biological materials, to emit fluorescence.

### [Reference Example 1]

In the image processing and measuring step for the evaluation step of Example 1, the correction of dividing the X value by the Y value was not performed. Instead, the average of the Y values for the first to fifth fields of view was calculated as the "uncorrected HER2 quantification value" for use in the evaluation of the HER2 expression level, and the coefficient of variation (CV) thereof was calculated.

### [Reference Example 2]

In the image processing and measuring step for the evaluation step of Example 2, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 3]

In the image processing and measuring step for the evaluation step of Example 3, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 4]

In the image processing and measuring step for the evaluation step of Example 4, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 5]

In the image processing and measuring step for the evaluation step of Example 5, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 6]

In the image processing and measuring step for the evaluation step of Example 6, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 7]

In the image processing and measuring step for the evaluation step of Example 7, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### [Reference Example 8]

In the image processing and measuring step for the evaluation step of Example 8, the correction of dividing the X value by the Y value was not performed as in Reference Example 1. Instead, the average of the Y values for the first to fifth fields of view was calculated, and the coefficient of variation of the uncorrected HER2 quantification value was calculated, as in Reference Example 1.

### <Results>

Table 1 shows the coefficients of variation CVs (%) obtained in Reference Examples 1 to 8, Examples 1 to 8, and Examples 9 to 16. The CVs obtained in Examples 1 to 8 are all at most 15% and lower than the CVs obtained in Reference Examples 1 to 8, which means that variations are smaller in Examples 1 to 8. It is apparent that the correction of the fluorescent label signal (the number of bright spots) from the target biological material by using the fluorescent label signal (brightness) from the reference biological material makes it possible to stably evaluate the expression level of the target biological material even when the samples are a collection of groups under different fixation conditions. The CVs obtained in Examples 9 to 16 are all at most 10% and lower than the CVs obtained in Examples 1 to 8, which means that variations are smaller in Examples 9 to 16. It is apparent that the use of a mixture (cocktail) of a plurality of reference biological materials makes it possible to evaluate the expression level of the target biological material more stably.

**[Table 1]**

| Cultured cells | CRL 1500 | SKBR3 | MDA-MB 4 53 | MDA-MB 175 | COLO 201 | MCF7 | Hela | MDA-MB 231 |
|---|---|---|---|---|---|---|---|---|
| Reference Examples (without correction) | 1 | 2 | 3 | 4 | 5 | 6 | 7 | 8 |
| CV | 20% | 19% | 25% | 25% | 21% | 18% | 22% | 20% |
| Examples (with correction) | 1 | 2 | 3 | 4 | 5 | 6 | 7 | 8 |
| CV (%) | 12% | 11% | 10% | 12% | 11% | 11% | 14% | 12% |
| Examples (with correction using cocktail) | 9 | 10 | 11 | 12 | 13 | 14 | 15 | 16 |
| CV (%) | 9% | 8% | 6% | 7% | 6% | 9% | 9% | 10% |

### [Comparative Examples]

### (C1) Sample preparation step

The sample preparation step was performed by the same procedure as in Example 1.

### (C2) Immunostaining steps

Immunostaining steps were performed according to the method described in the examples of Patent Literature 2 (JP 2008-286167 A), in which β-actin expressed in the cytoplasm was used as the reference biological material, and the target biological material HER2 was fluorescently labeled with a fluorescent dye itself rather than fluorescent dye-containing resin nanoparticles.

Specifically, the immunostaining steps were as follows.

### (C2-1) First immunostaining step (immunostaining of target biological material HER2)

### (C2-1-1) Primary reaction treatment

Using 1% BSA-containing PBS, 0.05 nM of an anti-HER2 rabbit monoclonal antibody 4B5 (Ventana) (reagent I) was prepared. The samples prepared in the step (1) were allowed to react at 4°C overnight by being immersed in the solution of the reagent I.

### (C2-1-2) Secondary reaction and fluorescent labeling treatment

Alexa Fluor 488-labeled anti-rabbit IgG antibody (A11008, Life Technologies) was diluted to 1/200 with 1% BSA-containing PBS to form a secondary reaction treatment liquid. After the primary reaction treatment, the samples were washed with PBS and then allowed to react at room temperature for 30 minutes by being immersed in the secondary reaction treatment liquid.

### (C2-2) Second immunostaining step (immunostaining of reference biological material β-actin)

### (C2-2-1) Primary reaction treatment

An anti-β-actin antibody (ab8229, Abcam) was diluted to 1/200 with 1% BSA-containing PBS to form a primary reaction treatment liquid. After the first immunostaining step, the samples were washed with PBS and then allowed to react at 4°C overnight by being immersed in the primary reaction treatment liquid.

### (C2-2-2) Secondary reaction and fluorescent labeling treatment

Alexa Fluor 647-labeled anti-mouse IgG antibody (A21236, Life Technologies) was diluted to 1/200 with 1% BSA-containing PBS to form a secondary reaction treatment liquid. After the primary reaction treatment, the samples were washed with PBS and then allowed to react at room temperature for 30 minutes by being immersed in the secondary reaction treatment liquid.

### (C2-3) Sample posttreatment step

After the first and second immunostaining steps, the samples were subjected to fixation and dehydration, in which immersion in pure ethanol for 5 minutes was performed four times . Subsequently, the samples were subjected to a cleaning treatment, in which immersion in xylene for 5 minutes was performed four times. Finally, the samples were subjected to a sealing treatment including putting a sealant Entellan New (Merck) on the samples and then covering each sample with a cover glass, so that samples for use in observation were obtained.

### (C3) Evaluation step

An evaluation step was performed according to the method described in the examples of Patent Literature 2 (JP 2008-286167 A), in which reference was made to the HER2 quantification value that was obtained by dividing the total fluorescence from the label on the target biological material HER2 by the total fluorescence from the label on the reference biological material β-actin. Specifically, the evaluation step was as follows.

### (C3-1) Observation and imaging step

In this step, a fluorescence microscope BX-53 (Olympus Corporation) was used for the irradiation with exciting light and the observation of emitted fluorescence, and a microscope digital camera DP73 (Olympus Corporation) attached to the fluorescence microscope was used to capture immunostaining images (at a magnification of × 400).

First, the sample was irradiated with exciting light for causing Alexa Fluor 488, which was used for the fluorescent labeling of the target biological material (HER2), to emit fluorescence, and the immunostaining image in that state was captured (the first immunostaining image) . In this process, the wavelength of the exciting light was set at 460 to 490 nm using an exciting light optical filter in the fluorescence microscope, and the wavelength of the fluorescence observed was set at 500 to 540 nm using a fluorescence optical filter. The observation with the fluorescence microscope and the intensity of the exciting light during the image capturing were such that an irradiation energy of 600 W/cm² was achieved at or near the center of the field of view. The exposure time during the image capturing was adjusted in the range where the brightness of the image was not saturated, and, for example, set at 800 msec.

Subsequently, the exciting light optical filter and the fluorescence optical filter were switched to other filters. The sample was irradiated with exciting light for causing Alexa Fluor 647, which was used for the fluorescent labeling of the reference biological material (β-actin), to emit fluorescence, and the immunostaining image in that state was captured (the second immunostaining image). In this process, the wavelength of the exciting light was set at 620 to 650 nm using an exciting light optical filter in the fluorescence microscope, and the wavelength of the fluorescence observed was set at 660 to 700 nm using a fluorescence optical filter. The observation with the fluorescence microscope and the intensity of the exciting light during the image capturing were such that an irradiation energy of 400 W/cm² was achieved at or near the center of the field of view. The exposure time during the image capturing was adjusted in the range where the brightness of the image was not saturated, and, for example, set at 800 msec.

After the first and second immunostaining images were captured in the same field of view, the same procedure was repeated in different fields of view. In total, the first and second immunostaining images were captured in three fields of view (first to third fields of view) for each sample.

### (C3-2) Image processing and measuring step

In this step, image processing was performed using image processing software ImageJ (open source).

The sum total (X') of the intensities of fluorescence emitted by the fluorescent material was determined using the first immunostaining image for the target biological material HER2. Similarly, the sum total (Y') of the degrees of brightness of fluorescence emitted by the fluorescent material was determined using the second immunostaining image for the reference biological material β-actin.

### (C3-3) Standardization step

Standardization was performed by dividing the X' value by the Y' value. After such a value was calculated for each of the first to third fields of view, the average of the resulting values was calculated as the "HER2 quantification value" for use in the evaluation of the HER2 expression level.

A coefficient of variation CV (= 100 × the standard deviation of the HER2 quantification values/the average of the HER2 quantification values) was calculated from the HER2 quantification values for 9 samples in total in the 1 hour-fixation treatment group (the number of samples n = 3), the 12 hour-fixation treatment group (n = 3), and the 96 hour-fixation treatment group (n = 3). The resulting CV was 22% and larger than the CV obtained in each example described above, which meant larger variations.

## Claims

1. A method for quantifying a target biological material expressed on a cell membrane in a pathological cell sample, the method comprising the steps of:
(1a) immunostaining the target biological material using a fluorescent material;
(1b) immunostaining a reference biological material using a fluorescent material different from the fluorescent material for the target biological material, wherein the reference biological material is different from the target biological material and constantly expressed on the cell membrane;
(2) using an immunostaining image for the target biological material and an immunostaining image for the reference biological material to identify the position of a fluorescent label signal corresponding to the target biological material on the cell membrane and to identify the position of a fluorescent label signal corresponding to the reference biological material on the cell membrane and to measure fluorescent label signals corresponding respectively to the target and the reference biological material on the cell membrane; and
(3) normalising the measured value of the fluorescent label signal corresponding to the target biological material on the cell membrane using the measured values of the fluorescent label signal corresponding to the reference biological material on the cell membrane, for a whole measurement region of the pathological cell sample or for each cell in the pathological cell sample, so that an index for quantifying an expression level is obtained.

2. The method according to claim 1, **characterized in that** the step (3) comprises the step of dividing the measured value of the fluorescent label signal corresponding to the target biological material in the cell membrane region by the measured value of the fluorescent label signal corresponding to the reference biological material to correct the measured value of the signal, so that an index for quantifying an expression level is obtained.

3. The method according to claim 1 or 2, **characterized by** further comprising a morphological observation staining step for performing morphological observation in a bright field before or after at least one of the steps (1a) and (1b).

4. The method according to claim 3, **characterized in that** the morphological observation staining step is hematoxylin-eosin staining.

5. The method according to claim 3 or 4, **characterized in that** in the step (3), the target biological material and the fluorescent label signal corresponding to the target biological material are measured at a wavelength of 550 nm to 630 nm.

6. The method according to any one of claims 1 to 5, **characterized in that** the fluorescent material for the target biological material is fluorescent dye-containing nanoparticles, and the fluorescent material for the reference biological material is a fluorescent dye.

7. The method according to claim 6, **characterized in that** the fluorescent dye-containing nanoparticles have an average particle size of 50 nm to 200 nm.

8. The method according to any one of claims 1 to 7, **characterized in that** there are two or more types of the reference biological materials.

9. The method according to any one of claims 1 to 8, **characterized in that** the reference biological material is one or more selected from the group consisting of ATPase, cadherin, cytokeratin, and EpCAM.

10. A kit for carrying out the method according to any one of claims 1 to 9 for quantifying a target biological material, the kit comprising:
an antibody and a fluorescent material for forming an immunostaining agent for the target biological material;
an antibody and a fluorescent material for forming an immunostaining agent for the reference biological material;
whereby, the target biological material is expressed on a cell membrane in a pathological cell sample;
the reference biological material is different from the target biological material and constantly expressed on the cell membrane; and
the immunostaining agent for the reference biological material comprises a fluorescent material different from the fluorescent material of the immunostaining agent for the target biological material.

## Patentansprüche

1. Verfahren zur Quantifizierung eines biologischen Zielmaterials, das auf einer Zellmembran in einer pathologischen Zellprobe exprimiert ist, wobei das Verfahren die Schritte aufweist:
(1a) Immunfärbung des biologischen Zielmaterials unter Verwendung eines fluoreszierenden Materials;
(1b) Immunfärbung eines biologischen Referenzmaterials unter Verwendung eines anderen fluoreszierenden Materials als das für das biologische Zielmaterial verwendete fluoreszierende Material, wobei das biologische Referenzmaterial sich von dem biologischen Zielmaterial unterscheidet und konstant auf der Zellmembran exprimiert ist;
(2) Verwendung eines Immunfärbungsbildes für das biologische Zielmaterial und eines Immunfärbungsbildes für das biologische Referenzmaterial zur Identifizierung der Position eines dem biologischen Zielmaterial auf der Zellmembran entsprechenden fluoreszierenden Markierungssignals und zur Identifizierung der Position eines dem biologischen Referenzmaterial auf der Zellmembran entsprechenden fluoreszierenden Markierungssignals und zur Messung von fluoreszierenden Markierungssignalen, die jeweils dem biologischen Zielmaterial und dem biologischen Referenzmaterial auf der Zellmembran entsprechen; und
(3) Normalisierung des Messwertes des dem biologischen Zielmaterial auf der Zellmembran entsprechenden fluoreszierenden Markierungssignals unter Verwendung der Messwerte des dem biologischen Referenzmaterial auf der Zellmembran entsprechenden fluoreszierenden Markierungssignals, für einen gesamten Messbereich der pathologischen Zellprobe oder für jede Zelle in der pathologischen Zellprobe, so dass ein Index zur Quantifizierung eines Expressionsniveaus erhalten wird.

2. Verfahren gemäß Anspruch 1, **dadurch gekennzeichnet, dass** der Schritt (3) den Schritt des Dividierens des Messwertes des dem biologischen Zielmaterial in dem Zellmembranbereich entsprechenden fluoreszierenden Markierungssignals durch den Messwert des dem biologischen Referenzmaterial entsprechenden fluoreszierenden Markierungssignals umfasst, um den Messwert des Signals zu korrigieren, so dass ein Index zur Quantifizierung eines Expressionsniveaus erhalten wird.

3. Verfahren gemäß Anspruch 1 oder 2, **dadurch gekennzeichnet, dass** es ferner einen Färbungsschritt zur morphologischen Betrachtung aufweist zur Durchführung einer morphologischen Betrachtung in einem Hellfeld vor oder nach zumindest einem der Schritte (1a) und 1(b).

4. Verfahren gemäß Anspruch 3, **dadurch gekennzeichnet, dass** der Färbungsschritt zur morphologischen Betrachtung aus der Färbung mit Hämatoxylin-Eosin besteht.

5. Verfahren gemäß Anspruch 3 oder 4, **dadurch gekennzeichnet, dass** in Schritt (3) das biologische Zielmaterial und das dem biologischen Zielmaterial entsprechende fluoreszierende Markierungssignal bei einer Wellenlänge von 550 nm bis 630 nm gemessen werden.

6. Verfahren gemäß irgendeinem der Ansprüche 1 bis 5, **dadurch gekennzeichnet, dass** das fluoreszierende Material für das biologische Zielmaterial aus fluoreszierenden Farbstoff enthaltenden Nanopartikeln besteht und das fluoreszierende Material für das biologische Referenzmaterial aus einer Fluoreszenzfarbe besteht.

7. Verfahren gemäß Anspruch 6, **dadurch gekennzeichnet, dass** die fluoreszierenden Farbstoff enthaltenden Nanopartikel eine durchschnittliche Partikelgröße von 50 nm bis 200 nm aufweisen.

8. Verfahren gemäß irgendeinem der Ansprüche 1 bis 7, **dadurch gekennzeichnet, dass** es zwei oder mehr Arten des biologischen Referenzmaterials gibt.

9. Verfahren gemäß irgendeinem der Ansprüche 1 bis 8, **dadurch gekennzeichnet, dass** das biologische Referenzmaterial aus einem oder mehr Elementen besteht, die ausgewählt sind aus der Gruppe, die ATPasen, Cadherin, Zytokeratin und EpCAM umfasst.

10. Set zur Durchführung des Verfahrens gemäß irgendeinem der Ansprüche 1 bis 9 zur Quantifizierung eines biologischen Zielmaterials, wobei das Set aufweist:
einen Antikörper und ein fluoreszierendes Material zur Bildung eines Immunfärbemittels für das biologische Zielmaterial;
einen Antikörper und ein fluoreszierendes Material zur Bildung eines Immunfärbemittels für das biologische Referenzmaterial;
wobei das biologische Zielmaterial auf einer Zellmembran in einer pathologischen Zellprobe exprimiert ist;
das biologische Referenzmaterial sich von dem biologischen Zielmaterial unterscheidet und konstant auf der Zellmembran exprimiert ist; und
das Immunfärbemittel für das biologische Referenzmaterial ein anderes fluoreszierendes Material aufweist als das fluoreszierende Material des Immunfärbemittels für das biologische Zielmaterial.

## Revendications

1. Procédé de quantification d'un matériau biologique cible exprimé sur une membrane de cellule dans un échantillon cellulaire pathologique, le procédé comprenant les étapes de :
(1a) immunocoloration du matériau biologique cible en utilisant un matériau fluorescent ;
(1b) immunocoloration d'un matériau biologique de référence en utilisant un matériau fluorescent différent du matériau fluorescent pour le matériau biologique cible, le matériau biologique de référence étant différent du matériau biologique cible et étant constamment exprimé sur la membrane de cellule ;
(2) utilisation d'une image d'immunocoloration pour le matériau biologique cible et une image d'immunocoloration pour le matériau biologique de référence pour identifier la position d'un signal marqueur fluorescent correspondant au matériau biologique cible sur la membrane de cellule et pour identifier la position d'un signal marqueur fluorescent correspondant au matériau biologique de référence sur la membrane de cellule et pour mesurer des signaux marqueurs fluorescents correspondant respectivement au matériau biologique cible et au matériau biologique de référence sur la membrane de cellule ; et
(3) normalisation de la valeur mesurée du signal marqueur fluorescent correspondant au matériau biologique cible sur la membrane de cellule en utilisant les valeurs mesurées du signal marqueur fluorescent correspondant au matériau biologique de référence sur la membrane de cellule, pour une zone entière de mesure de l'échantillon cellulaire pathologique ou pour chaque cellule dans l'échantillon cellulaire pathologique, de sorte qu'un index pour quantifier un niveau d'expression est obtenu.

2. Procédé selon la revendication 1, **caractérisé en ce que** l'étape (3) comprend l'étape de diviser la valeur mesurée du signal marqueur fluorescent correspondant au matériau biologique cible dans la région de la membrane de cellule par la valeur mesurée du signal marqueur fluorescent correspondant au matériau biologique de référence pour corriger la valeur mesurée du signal, de sorte qu'un index pour quantifier un niveau d'expression est obtenu.

3. Procédé selon la revendication 1 ou la revendication 2, **caractérisé en ce qu'**il comprend en outre une étape de coloration pour l'observation morphologique pour effectuer une observation morphologique dans un champ clair avant ou après au moins une des étapes (1a) et (1b).

4. Procédé selon la revendication 3, **caractérisé en ce que** l'étape de coloration pour l'observation morphologique comprend une coloration à l'hématoxyline-éosine.

5. Procédé selon la revendication 3 ou la revendication 4, **caractérisé en ce que** dans l'étape (3) le matériau biologique cible et le signal marqueur fluorescent correspondant au matériau biologique cible sont mesurés à une longueur d'ondes comprise entre 550 nm et 630 nm.

6. Procédé selon l'une quelconque des revendications 1 à 5, **caractérisé en ce que** le matériau fluorescent pour le matériau biologique cible consiste en des nanoparticules contenant un colorant fluorescent, et le matériau fluorescent pour le matériau biologique de référence est un colorant fluorescent.

7. Procédé selon la revendication 6, **caractérisé en ce que** les nanoparticules contenant un colorant fluorescent ont une taille moyenne de particule comprise entre 50 nm et 200 nm.

8. Procédé selon l'une quelconque des revendications 1 à 7, **caractérisé en ce qu'**il y a deux types ou plus de matériaux biologiques de référence.

9. Procédé selon l'une quelconque des revendications 1 à 8, **caractérisé en ce que** le matériau biologique de référence est un ou plusieurs élément(s) sélectionné(s) dans le groupe composé de ATPase, de cadhérine, de cytokératine et de EpCAM.

10. Kit pour effectuer le procédé selon l'une quelconque des revendications 1 à 9 pour quantifier un matériau biologique cible, le kit comprenant :
un anticorps et un matériau fluorescent pour former un agent d'immunocoloration pour le matériau biologique cible ;
un anticorps et un matériau fluorescent pour former un agent d'immunocoloration pour le matériau biologique de référence ;
dans lequel le matériau biologique cible est exprimé sur une membrane de cellule dans un échantillon cellulaire pathologique ;
le matériau biologique de référence est différent du matériau biologique cible et est constamment exprimé sur la membrane de cellule ;
et
l'agent d'immunocoloration pour le matériau biologique de référence comprend un matériau fluorescent différent du matériau fluorescent de l'agent d'immunocoloration pour le matériau biologique cible.
